# EUROPEAN PATENT SPECIFICATION

(11) **EP 1 508 321 B1**
(45) Date of publication and mention of the grant of the patent: **23.05.2007**
(21) Application number: 04019518.2
(22) Date of filing: 17.08.2004
(51) Int. Cl.: A61K 6/00, A61K 6/08

(54) **Dental adhesive composition**
Dentalklebstoffzusammensetzung
Composition adhésive dentaire

(30) Priority: 22.08.2003 JP 2003298363
(43) Date of publication of application: 23.02.2005
(73) Proprietor: DENTSPLY-SANKIN K.K., Ohtawara-shi, Tochigi (JP)
(72) Inventor: Anzai, Misaki, Asao-ku Kawasaki-shi Kanagawa (JP); Kawaguchi, Motoki, Nishinasunomachi Nasu-gun Tochigi (JP)
(74) Representative: Müller-Boré & Partner Patentanwälte

(56) References cited:
- EP-A- 0 282 280
- EP-A- 1 287 805
- WO-A-98/49997
- US-A- 5 089 051
- DATABASE WPI Section Ch, Week 199411 Derwent Publications Ltd., London, GB; Class A14, AN 1994-089230 XP002304388 & JP 06 040836 A (MITSUI PETROCHEM IND CO LTD) 14 February 1994 (1994-02-14) & JP 06 040836 A (MITSUI PETROCHEM IND CO LTD) 14 February 1994 (1994-02-14)
- DATABASE WPI Section Ch, Week 199411 Derwent Publications Ltd., London, GB; Class A14, AN 1994-089231 XP002304389 & JP 06 040837 A (MITSUI PETROCHEM IND CO LTD) 15 February 1994 (1994-02-15) & JP 06 040837 A (MITSUI PETROCHEM IND CO LTD) 15 February 1994 (1994-02-15)
- DATABASE WPI Section Ch, Week 200382 Derwent Publications Ltd., London, GB; Class A41, AN 2003-882846 XP002304390 & JP 2003 201286 A (MITSUI CHEM INC) 18 July 2003 (2003-07-18)
- DATABASE WPI Section Ch, Week 197430 Derwent Publications Ltd., London, GB; Class A14, AN 1974-54251V XP002304391 & JP 48 089947 A (TOA GOSEI CHEM IND LTD) 24 November 1973 (1973-11-24)

## Description

The present invention relates to a dental adhesive composition to be used for dental restoration, and more particularly, to a dental adhesive composition hardenable by polymerization, which can give strong adhesion between tooth substances and restorative materials with excellent workability in handling even in a wet condition or even in an atmosphere containing air (or oxygen).

As the materials to be used for the restoration of defects of tooth substances, restorative filling materials called composite resins have been known, which comprise polymerizable monomers and inorganic fillers. When restorations made of ceramics or metals are bonded to teeth, adhesives called resin cements have been used, which comprise acidic group containing polymerizable monomers and inorganic fillers. In some cases, bonding agents or primers may be used to give strong adhesion between the composite resins or resin cements and the teeth.

In such dental materials, (meth) acrylate monomers have been widely used, which can be hardened by polymerization with the use of a polymerization initiator of the redox type, which is composed of a peroxide and a reducing agent, or with the use of a photopolymerization initiator which can generate a radical by irradiation of light.

For example, Japanese Patent Laid-open (Kokai) Publications Nos. 6-40836 and 6-40837 disclose dental adhesive compositions hardenable by polymerization with a radical polymerization initiator such as a peroxide, wherein the compositions comprise a (meth)acrylate monomer having at least one hydroxyl group in one molecule, a (meth) acrylate monomer having at least one carboxyl group in one molecule, a multifunctional (meth) - acrylate monomer having two (meth)acryloyl groups in one molecule, and an aromatic amino compound having at least one carboxyl group in one molecule, or a metal salt thereof.

The dental adhesive compositions described above have their own excellent properties, but they much need to be improved. More specifically, adhesive compositions hardenable by polymerization with a radical polymerization initiator may be inhibited in their polymerization by oxygen in air or by dissolved oxygen in a paste, and in particular, the percentage of polymerization on the surface coming in contact with air may significantly be lowered in some cases. In addition, for example, when resin cements for adhesive bonding are used to bond restorations, resin cement layers (or cement lines) of about 50 to 100 µm in thickness are formed in gaps between the restorations and the teeth, in which the cement lines are exposed to air so that the percentage of polymerization is consequently lowered, resulting in a fear about the possible change of color in the cement lines or the possible dissolution of the cement lines in saliva. Furthermore, it is impossible to obtain sufficient mechanical strength, which brings a shortage of bond strength, resulting in a fear about the possible detachment of the restorations or the possible secondary caries.

On the other hand, it cannot be said that ordinary resin cements have sufficient adhesion properties to tooth substances; therefore, a troublesome procedure is needed for adhesive bonding, in which a low-viscosity bonding agent (or a adhesive primer) is first applied to the tooth surface and a restorative material is then allowed to adhere to the teeth with a resin cement.

The present invention has been completed by taking particular note of the above problems. It is an object of the present invention to provide a dental adhesive composition which can quickly be hardened even in the presence of oxygen without using a radical polymerization initiator such as a peroxide or without using a photopolymerization initiator, to give a hardened material, and it is another object of the present invention to provide a dental adhesive composition which can give direct and strong adhesion between tooth substances and restorative materials without using a bonding agent (or an adhesive primer).

Thus the present invention, which can solve the above problems, provides a dental adhesive composition comprising:
(A) a carboxylic acid compound having a (meth)-acryloyl group and a carboxyl group, both of which are attached to an aromatic ring, in an amount not smaller than 10% by weight and not greater than 65% by weight;
(B) a bisphenol A derivative having two (meth)-acryloyl groups, in an amount equal to 0% by weight or not greater than 45% by weight; and
(C) a hydroxyalkyl (meth) acrylate in an amount not smaller than 30% by weight and not greater than 70% by weight;
   the amounts of the ingredients (A), (B), and (C) being all based on the total weight of the ingredients (A), (B), and (C);
   the composition further comprising:
(D) a (meth)acrylate derivative having an acid group, in an amount not smaller than 10 parts by weight and not greater than 40 parts by weight, based on 100 parts by the total weight of the ingredients (A), (B), and (C); and
(E) at least one polymerization initiator selected from the group consisting of aromatic amines, aliphatic amines, and aromatic sulfinic acids, in an amount not smaller than 1 part by weight and not greater than 11 parts by weight, based on 100 parts by the total weight of the ingredients (A), (B), and (C);
   the composition being substantially free from any radical polymerization initiator.

The dental adhesive composition of the present invention may further comprises (F) a filler for filling reinforcement, in which case the filler (F) is preferably added in an amount not greater than 350 parts by weight, more preferably in an amount not smaller than 50 parts by weight and not greater than 300 parts by weight, based on 100 parts by the total weight of the ingredients (A), (B), and (C).

The dental adhesive composition of the present invention may further comprise (G) an oxetane resin, in which case the oxetane resin (G) is preferably added in an amount not greater than 60 parts by weight, more preferably in an amount not smaller than 20 parts by weight and not greater than 40 parts by weight, based on 100 parts by the total weight of the ingredients (A), (B), and (C).

In the dental adhesive composition of the present invention, specific examples of the carboxylic acid compound (A) are 2-(meth)acryloyloxyethyl hydrogenphthalate and 4-(meth)acryloyloxyethyl hydrogenphthalate, and specific examples of the bisphenol A derivative (B) are 2,2-bis[4-(3-methacryloyloxy-2-hydroxypropoxy)-phenyl]propane and 2,2-bis[(4-methacryloyloxypolyethoxy)phenyl]propane.

In the dental adhesive composition of the present invention, inorganic fine particles and/or organic fine particles can be used as the filler (F), usually used being silica fine particles and most preferred being silica fine particles having an average particle diameter not smaller than 0.2 µm and not greater than 5 µm.

In the dental adhesive composition of the present invention, specific examples of the oxetane resin (G) are 3-(methacryloyloxy(lower alkyl))-3-(lower alkyl)-oxetane, tetraphthalate-bisoxetane, and biphenylene-bisoxetane, all of which can be used alone or in combination.

The dental adhesive composition of the present invention contains (A) a carboxylic acid compound having a (meth)acryloyl group and a carboxyl group, both of which are attached to an aromatic ring. The carboxylic acid compound (A) is an essential ingredient to an improvement in the mechanical properties of a hardened material, which is obtained by copolymerization with other monomers contained in the dental adhesive composition; an exhibition of the effect of improving adhesion properties by the carboxylic group attached to the aromatic ring; and an improvement in water resistance by the aromatic ring providing the hardened material with a hydrophobic group.

Typical examples of the carboxylic acid compound (A) are 2-methacryloyloxyethyl hydrogenphthalate and 4-methacryloyloxyethyl hydrogenphthalate. These compounds can be used alone or in combination. Particularly preferred is 2-methacryloyloxyethyl hydrogenphthalate.

The amount of the carboxylic acid compound (A) is not smaller than 10% by weight and not greater than 65% by weight, based on the total weight of the ingredients (A), (B), and (C). If the amount of the carboxylic acid compound (A) is smaller than 10% by weight or greater than 65% by weight, hardening reaction by polymerization proceeds slowly, thereby decreasing working efficiency in adhesive bonding as well as giving a tendency to the shortage of bond strength. The preferred amount of the carboxylic acid compound (A) is not smaller than 10% by weight and not greater than 55% by weight, more preferably not smaller than 15% by weight and not greater than 50% by weight, based on the total weight of the ingredients (A), (B), and (C).

The bisphenol A derivative (B) having two (meth) - acryloyl groups can be expected to have the effect of increasing the viscosity of a paste and can be effective as a crosslinking agent to be reacted with the monomers contained in the composition for improving the mechanical properties of a hardened material as well as for exhibiting an action to improve water resistance. The bisphenol A derivative (B), although it may desirably be added in an appropriate amount, is not an essential ingredient in the dental adhesive composition of the present invention. For example, when the carboxylic acid compound (A) is added in a greater amount and the hydroxylalkyl(meth)acrylate (C) is added in a smaller amount, or when the filler (F) is added in a greater amount, the objects of the present invention can be attained without using the bisphenol A derivative (B) . The bisphenol A derivative (B) is, however, preferably added in an amount not smaller than 1% by weight, based on the total weight of the ingredients (A), (B), and (C), because the addition of the bisphenol A derivative (B) in an appropriate amount gives better workability in mixing and better workability in adhesive bonding of a dental adhesive composition, so that the resulting dental adhesive composition can exhibit more excellent performance, for example, from a clinical point of view, when used for the bonding of restorations. However, if the bisphenol A derivative (B) is added in an excessive amount, the viscosity of a paste is much increased, resulting in various significant drawbacks, for example, a deterioration of workability in mixing and workability in adhesive bonding; a difficulty to obtain a desired bond strength; and a lowering of polymerization reactivity to prolong setting time. Therefore, it is better to keep the amount of the bisphenol A derivative not greater than 45% by weight, preferably not greater than 40% by weight, based on the total weight of the ingredients (A), (B), and (C).

The bisphenol A derivative (B) is not particularly limited to specific compounds, so long as it is a bisphenol A derivative having two (meth)acryloyl groups. Preferred examples are 2,2-bis[4-(3-methacryloyloxy-2-hydroxypropoxy)phenyl]propane and 2,2-bis[(4-methacryloyloxypolyethoxy)phenyl]propane. Particularly preferred is 2,2-bis[4-(3-methacryloyloxy-2-hydroxy-propoxy)phenyl]propane.

When the bisphenol A derivative (B) is not used in the dental adhesive composition of the present invention, the amounts of the carboxylic acid compound (A) and the hydroxyalkyl (meth)acrylate (C) are expressed as their respective percentages by weight, based on the total weight of the carboxylic acid compound (A) and the hydroxyalkyl (meth)acrylate (C).

The hydroxyalkyl (meth)acrylate (C) is an essential ingredient to a lowering in the viscosity of a paste as a dental adhesive composition to improve workability in mixing and workability in adhesive bonding; a flexibility given to a polymerized material; an effective action as a solvent of the polymerization initiator (E); and an improvement in bond strength as a cement. The hydroxyalkyl (meth) acrylate (C) is preferably added in an amount not smaller than 30% by weight and not greater than 70% by weight, based on the total weight of the ingredients (A), (B), and (C). If the amount of the hydroxyalkyl (meth)acrylate (C) is smaller than 30% by weight, the resulting dental adhesive composition comes to have too high a viscosity to deteriorate workability in handling. In contrast, if the amount of the hydroxylalkyl (meth)acrylate (C) is greater than 70% by weight, it is not preferred because of a deterioration of workability in mixing and workability in adhesive bonding as well as a deterioration of the physical properties of a hardened material which is obtained by polymerization. Totally taking into consideration the workability in handling, workability in mixing and in adhesive bonding of an adhesive composition, as well as the physical properties of a hardened material, the hydroxylalkyl (meth) acrylate (C) is preferably added in an amount not smaller than 30% by weight and not greater than 60% by weight, based on the total weight of the ingredients (A), (B), and (C). The most versatile example of the hydroxyalkyl (meth)acrylate (C) is a hydroxylethyl methacrylate.

In the present invention, besides the polymerizable ingredients (A), (B), and (C) described above, the (meth)acrylate derivative (D) having an acid group is desirably added in an appropriate amount. Examples of the ingredient (D) are (meth)acrylate compounds each having at least one carboxyl group or at least one phosphoric acid group. Preferred specific examples are 11-methacryloyloxy-1,1-undecanedicarboxylic acid, 4-acryloyloxyethyltrimellitic acid, 10-methacryloyloxydecyl hydrogenphosphate, and 2-methacryloyloxy-ethylphenyl phosphate. The acid portions contained in their molecules can give strong adhesion between restorations and tooth substances as an adhesive composition to exhibit an action to improve adhesive strength.

The action of the (meth)acrylate derivative (D) is effectively exhibited by addition in an amount not smaller than 10 parts by weight, based on 100 parts by the total weight of the ingredients (A), (B), and (C). If the amount of the ingredient (D) is greater than 40 parts by weight, it results in a deterioration of workability in mixing and workability in adhesive bonding as well as a tendency of adhesive strength to be lowered even more. The amount of the ingredient (D) is preferably not smaller than 10 parts by weight and not greater than 30 parts by weight, based on 100 parts by the total weight of the ingredients (A), (B), and (C).

When the bisphenol A derivative (B) is not used in the dental adhesive composition of the present invention, the amount of the (meth)acrylate derivative (D) is expressed as its parts by weight, based on 100 parts by the total weight of the carboxylic acid compound (A) and the hydroxyalkyl (meth)acrylate (C).

In the present invention, to effect hardening reaction by polymerization of a polymerizable monomer system including the ingredients (A) to (D) even in a wet condition or even in the presence of air, substantially no radical polymerization initiator is used, that is, the dental adhesive composition of the present invention is substantially free from any radical polymerization initiator, but at least one selected from the group consisting of aromatic amines, aliphatic amines, and aromatic sulfinic acids is used as the polymerization initiator (E). The terms "aromatic amines" and "aliphatic amines" refer to aromatic tertiary amines and aliphatic tertiary amines, respectively. Preferred examples are N,N-dimethyl-para-toluidine or paratolyldiethanolamine. Examples of the aromatic sulfinic acids are para-toluenesulfinic acid and salts thereof.

These polymerization initiators (E) can be used alone or in combination to exhibit the function as a polymerization catalyst for the polymerizable monomer system including the ingredients (A) to (D), particularly even in a wet condition or even in an atmosphere containing oxygen, thereby giving a hardened material of high strength with excellent workability in handling .

The kind and amount of the polymerization initiator (E) may be determined according to the setting time and bond strength required for the adhesive composition as well as the physical properties of a hardened material obtained by polymerization. The preferred amount of the polymerization initiator (E) is not smaller than 1 part by weight and not greater than 11 parts by weight, based on 100 parts by the total weight of the ingredients (A), (B), and (C) . If the amount of the polymerization initiator (E) is smaller than 1 part by weight, the shortage of hardening by polymerization causes a deterioration of working efficiency as well as a tendency to deteriorate the physical properties of the hardened material. In contrast, if the amount of the polymerization initiator (E) is greater than 11 parts by weight, hardening reaction proceeds so fast that workability in mixing may be deteriorated and the hardened material may cause a deterioration of appearance by coloration. For the purpose of surely preventing the coloration of the hardened material, it is desirable to use an aromatic sulfinic acid in an amount not smaller than 1 part by weight and not greater than 5 parts by weight, based on 100 parts by the total weight of the ingredients (A), (B), and (C).

When the bisphenol A derivative (B) is not used in the dental adhesive composition of the present invention, the amount of the polymerization initiator (E) is expressed as its parts by weight, based on 100 parts by the total weight of the carboxylic acid compound (A) and the hydroxyalkyl (meth)acrylate (C).

In the present invention, the filler (F) can further be added as another ingredient to the composition. The filler (F) is an effective ingredient for improving the strength of a hardened material as a filling reinforcement. Examples of the filler (F) are inorganic fine particles such as those made of silica (SiO₂), titanium oxide (TiO₂), aluminum oxide (Al₂O₃), aluminosilicate glass (Al₂O₃·SiO₂), or fluorosilicate glass; and organic fine particles such as those made of cross-linked polymers or cross-linked copolymers. These fine particles can be used alone or in combination.

The filler (F), although it is not an essential ingredient in the dental adhesive composition of the present invention, may desirably be added in an amount not smaller than 10 parts by weight, more preferably not smaller than 50 parts by weight, based on 100 parts by the total weight of the ingredients (A), (B), and (C), for the purpose of further improving, through the filling reinforcement action as described above, the strength of the hardened material obtained by polymerization. However, if the filler (F) is added in a too great amount, the amounts of the polymerizable monomer ingredients have a tendency to become relatively insufficient, thereby causing poor hardening; the hardened material has more of a tendency to deteriorate the physical properties thereof; and the viscosity of the composition becomes increased to deteriorate workability in handling. Therefore, the amount of the filler (F) is to be adjusted to not greater than 350 parts by weight, more preferably not greater than 300 parts by weight, based on 100 parts by the total weight of the ingredients (A), (B), and (C).

When the bisphenol A derivative (B) is not used in the dental adhesive composition of the present invention, the amount of the filler (F) is expressed as its parts by weight, based on 100 parts by the total weight of the carboxylic acid compound (A) and the hydroxyalkyl (meth)acrylate (C).

A further useful ingredient which can be added to the dental adhesive composition of the present invention is the oxetane resin (G). Examples of the oxetane resin (G) are 3-(methacryloyloxymethyl)-3-methyloxetane, 3-(methacryloyloxymethyl)-3-ethyloxetane, 3-(methacryloyloxymethyl)-3-butyloxetane, 3-(methacryloyloxymethyl)-3-hexyloxetane,tetraphthalate-bisoxetane, and biphenylene-bisoxetane. The oxetane resin (G), although it is not an essential ingredient in the dental adhesive composition of the present invention, may exhibit an action of reducing shrinkage caused by the polymerization of the adhesive composition and may have an effective action to further improve bond strength because a rigid hardened material can easily be obtained, when added in an appropriate amount.

The amount of the oxetane resin (G), which is preferred for the purpose of making the oxetane resin (G) effectively exhibit the actions described above, may be not smaller than 5 parts by weight and not grater than 60 parts by weight, more preferably not smaller than 10 parts by weight and not greater than 50 parts by weight, based on 100 parts by the total weight of the ingredients (A), (B), and (C). If the amount of the oxetane resin (G) is smaller than 5 parts by weight, the action of the oxetane resin (G) to be attained by addition is substantially not observed. In contrast, if the amount of the oxetane resin (G) is greater than 60 parts by weight, there arises a fear to have such adverse effects that the viscosity of the resulting paste becomes much increased to extremely deteriorate workability in mixing and workability in adhesive bonding, and that it becomes difficult to obtain a uniformly polymerized material.

When the bisphenol A derivative (B) is not used in the dental adhesive composition of the present invention, the amount of the oxetane resin (G) is expressed as its parts by weight, based on 100 parts by the total weight of the carboxylic acid compound (A) and the hydroxyalkyl (meth)acrylate (C).

The materials to be used for the dental adhesive composition of the present invention are as described above. For the purpose of obtaining a uniform adhesive composition by mixing these materials, the use of an appropriate solvent or dispersion medium is preferred, and in particular, the combined use of water and a water-soluble organic solvent in appropriate amounts is desired. Examples of the water-soluble organic solvent are lower alcohols such as methanol, ethanol, and propyl alcohol; and ketones such as acetone and methyl acetone. Particularly preferred are ethanol, acetone, and mixtures thereof.

The amount of water or a water-soluble organic solvent, although it depends on the kinds and amounts of the ingredients (A) to (G), is typically not smaller than 0.1% by weight and not greater than 20% by weight, more generally not smaller than 1% by weight and not greater than 10% by weight, for the organic solvent, and typically not smaller than 0.1% by weight and not greater than 15% by weight, more preferably not smaller than 0.2% by weight and not greater than 10% by weight, for water, based on the total weight of the dental adhesive composition.

The dental adhesive composition of the present invention can be prepared by uniformly mixing the above materials in specific amounts. For the purpose of improving the stability in storage and the workability in handling of the dental adhesive composition, the following method of preparation and use is preferred because it becomes possible to ensure not only further excellent workability in mixing, and further excellent workability in handling, but also higher bond strength: a paste obtained by dissolving or dispersing the ingredients (A), (D), and (E), and if necessary, the ingredients (F) and (G), in a given solvent, and a paste obtained by dissolving or dispersing the ingredients (B) and (C), and if necessary, the ingredient (F), in a given solvent are prepared separately, and these pastes are used in the form of a two-component mixing type in which they are mixed just before use. In this regard, however, the above method of preparation is described only as a typical example, and it can be, of course, appropriately changed, altered, or modified according to the kinds and amounts of ingredients to be added; the condition of an atmosphere in adhesive bonding; and the desired strength of a hardened material.

### Examples

The present invention will be further described by the following examples, comparable examples, and reference examples; however, the present invention is not limited to these examples, and can be put into practice by making appropriate changes, alterations, or modifications within the range adapted to the purports described hereinbefore and hereinafter, all of the changes, alterations, and modifications being also included in the technical scope of the present invention.

Various dental adhesive compositions were prepared by mixing the materials in the respective amounts (percentages by weight, based on the total weight of the ingredients (A), (B), and (C), for the ingredients (A) to (C), and parts by weight, based on 100 parts by the total weight of the ingredients (A), (B), and (C), for the ingredients (D) to (G)) shown in Table 1 by the method of preparation described below, and then examined for bond strength and workability in mixing and in adhesive bonding by the test method described below. The test results are shown in Table 1.

### Method of Preparation

Taking into consideration the stability in storage of a dental adhesive composition, two types of pastes, i.e., pastes A and B, were prepared, and both were mixed to give an adhesive composition.

Paste A was prepared by adding N, N-dimethyl-paratoluidine (hereinafter abbreviated as N,N-DMPT) and/or para-tolyldiethanol amine (hereinafter abbreviated as p-TDEA) and/or sodium para-toluenesulfinate (hereinafter abbreviated as p-TSNa) in a prescribed amount (s) to 2-methacryloyloxyethyl hydrogenphthalate (hereinafter abbreviated as MEHP) in a prescribed amount; further adding thereto 11-methacryloyloxy-1,1-undecane-dicarboxylic acid (hereinafter abbreviated as MAC-10) and/or 10-methacryloyloxydecyl hydrogenphosphate (hereinafter abbreviated as MDP) in a prescribed amount(s); further adding thereto biphenylene-bisoxetane (hereinafter abbreviated as OXBP) in a prescribed amount: and then mixing the thus obtained mixture with silica (SiO₂) fine particles having an average particle diameter of 1.5 µm in an appropriate amount, taking into consideration the viscosity and workability in handling of the resulting paste.

On the other hand, paste B was prepared by mixing 2,2-bis[4-(3-methacryloyloxy-2-hydroxypropoxy)phenyl]propane (hereinafter abbreviated as Bis-GMA) and hydroxyethyl methacrylate (hereinafter abbreviated as HEMA) in prescribed amounts; and then mixing the thus obtained mixture with silica (SiO₂) fine particles having an average particle diameter of 1.5 µm in an appropriate amount, taking into consideration the viscosity and workability in handling of the resulting paste.

Pastes A and B obtained in such a manner as described above were mixed in a ratio by weight of 1 : 1 to give a dental adhesive composition.

### Bond Strength Test

The dental adhesive compositions obtained above were used to measure bond strength with the dentines of bovine teeth according to the following method.

For test teeth, mandibular anterior teeth of bovines (two to three years old) were extracted, except deficiently calcified teeth, root incomplete teeth, and significantly damaged teeth, and the extracted teeth were then immediately subjected to pulpectomy, followed by storage in a refrigerator. When the test teeth were used, the roots of teeth were removed, and only the tooth crowns were embedded in a self-curing resin (manufactured by DENTSPLY-SANKIN K.K.), the labial surfaces of which were grinded with a piece of carborundum paper #600 so as to give the dentines of 6 to 8 mm in diameter, followed by ultrasonic washing for 5 minutes and then drying with an air syringe.

To the surface of each adherend was attached a seal (of 50 µm in thickness) with a hole of 4 mm in diameter to define the adherend area, which was regarded as the adherend surface. To this adherend surface was applied a paste of the test dental adhesive composition, which was covered with a piece of aluminum foil and slightly pressed with a finger, followed by keeping in a thermo-hygrostat at a temperature of 37°C and at a relative humidity of 95% for 1 hour to effect hardening. The piece of aluminum foil was then removed, and a transparent tube (made of polypropylene) of 5 mm in diameter and 6 mm in height was placed on the adherend surface and filled with a chemically polymerizable composite resin (manufactured by DENTSPLY-SANKIN K.K.), in the top surface of which was embedded a hook for the measurement of bond strength, to give a test piece for the measurement of tensile bond strength.

The bond strength was measured with a universal testing machine (type 5567, manufactured by Instron Corporation) operating at a cross head speed of 1 mm/min. in a thermo-hygrostatic chamber at a temperature of 23°C and at a humidity of 50 ± 5%. Five test pieces were used and evaluated with an average of measured values in MPa for these test pieces and a standard deviation (S.D.) of the average.

### Workability in Mixing and in Adhesive Bonding

On mixing paper was weighed 0.5 g of each of pastes A and B, which were mixed with a dental plastic spatula for 30 seconds. At this time, if the cement paste had adequate fluidity, good wettability with the dentine adherend, and viscosity in such a degree that it was not carried away from the adherend surface, workability in mixing and in adhesive bonding was evaluated as "good". In contrast, when the cement paste had high viscosity, causing difficulty in mixing for 30 seconds, and poor wettability with the adherend surface, causing difficulty in adhesive bonding, and when the cement paste had too high fluidity in mixing to give adequate viscosity, so that the cement paste was carried away from the adherend surface, causing difficulty in adhesive bonding, workability in mixing and in adhesive bonding was evaluated as "bad" in both cases.

**TABLE 1**

| Experiment No. | 1 | 2 | 3 | 4 | 5 | 6 | 7 | 8 | 9 | 10 | 11 | 12 | 13 | 14 | 15 | 16 | 17 | 18 | 19 |
|---|---|---|---|---|---|---|---|---|---|---|---|---|---|---|---|---|---|---|---|
| (A) MEHP | 20 | 10 | 35 | 20 | 60 | 50 | 65 | 8 | 10 | 10 | 5 | 75 | 50 | 65 | 20 | 35 | 35 | 20 | 35 |
| (B) Bis-GMA | 30 | 45 | 5 | 10 | - | 20 | - | 30 | 50 | 5 | 10 | - | 35 | 10 | 30 | 5 | 5 | 10 | 5 |
| (C) HEMA | 50 | 45 | 60 | 70 | 40 | 30 | 35 | 62 | 40 | 85 | 85 | 25 | 15 | 25 | 50 | 60 | 60 | 70 | 60 |
| (D) MAC-10 | 30 | 5 | 20 | 10 | 20 | 10 | 5 | 30 | 5 | 20 | 10 | 20 | 10 | 5 | 5 | 20 | 30 | 10 | 20 |
| MDP | - | 10 | 15 | - | 20 | 30 | 35 | - | 10 | 15 | - | 20 | 30 | 35 | - | 30 | 15 | - | 15 |
| (E) N,N-DMPT | 2 | - | 1 | 1 | 1 | - | 1 | 2 | - | | 1 | 1 | - | 1 | 2 | 1 | 1 | 1 | 1 |
| p-TDEA | 4 | 6 | 2 | - | 2 | 6 | 1 | 4 | 6 | 2 | - | 2 | 6 | 1 | 4 | 2 | 2 | - | 2 |
| p-TSNa | 5 | 1 | 2 | 4 | 3 | 2 | 5 | 5 | 1 | 2 | 4 | 3 | 2 | 5 | 5 | 2 | 2 | 4 | 2 |
| (F) SiO₂ | 50 | 100 | - | 350 | 100 | 50 | 250 | 50 | 100 | - | 350 | 100 | 50 | 250 | 50 | - | - | 400 | - |
| (G) OXBP | 40 | - | - | 30 | 20 | 60 | 60 | 40 | - | - | 30 | 20 | 60 | 60 | 40 | - | - | - | 65 |
| Bond strength (Mpa) | 11.0 | 12.0 | 15.2 | 10.0 | 10.2 | 14.2 | 8.0 | 7.0 | 6.5 | 7.5 | 5.4 | 6.6 | 5.5 | 4.0 | 5.0 | 5.0 | 6.8 | 5.1 | 4.1 |
| Standard deviation | 0.8 | 1.1 | 1.4 | 0.6 | 1.0 | 1.3 | 0.8 | 0.6 | 0.6 | 1.1 | 0.6 | 0.5 | 1.0 | 0.2 | 0.4 | 0.4 | 0.6 | 0.5 | 0.4 |
| Workability in mixing and in adhesive bonding | good | good | good | good | good | good | good | bad | bad | bad | good | bad | bad | bad | bad | bad | bad | bad | bad |

In Table 1, Experiments Nos. 1 to 7 correspond to examples complying with all the essential requirements (claim 1) established in the present invention, in which all cases high bond strength and good workability in mixing and in adhesive bonding were obtained.

In contrast, Experiments Nos. 8 to 17 correspond to comparative examples failing to comply with any one of the essential requirements (claim 1) established in the present invention, in which all cases at least one of bond strength and workability in mixing and in adhesive bonding was bad as described below, and which cannot be said to attain the objects of the present invention.

Furthermore, Experiments 18 and 19 correspond to reference examples complying with all the essential requirements (claim 1) established in the present invention, but failing to comply with either of the optional requirements (claim 2 or 3) established in the present invention, in which both cases low bond strength and bad workability in mixing and in adhesive bonding were obtained as described below, and which cannot be said to attain the objects of the present invention.

Experiment No. 8: workability in mixing and in adhesive bonding was deteriorated and bond strength was relatively low, because the amount of the ingredient
(A) was insufficient.
   Experiment No. 9: workability in mixing and in adhesive bonding was deteriorated and bond strength was relatively low, because the amount of the ingredient
(B) was too great.
   Experiment No. 10: workability in mixing and in adhesive bonding was deteriorated and bond strength was relatively low, because the amount of the ingredient
(C) was too great.
   Experiment No. 11: workability in mixing and in adhesive bonding was good, but bond strength was extremely low, because the amount of the ingredient (A) was insufficient and the amount of the ingredient (C) was too great.
   Experiment No. 12: workability in mixing and in adhesive bonding was deteriorated and bond strength was low, because the amount of the ingredient (A) was too great and the amount of the ingredient (C) was insufficient.
   Experiments Nos. 13 and 14: workability in mixing and in adhesive bonding was deteriorated and bond strength was low, because the amount of the ingredient
(C) was insufficient.
   Experiment No. 15: workability in mixing and in adhesive bonding was deteriorated and bond strength was low, because the amount of the ingredient (D) was insufficient.
   Experiments Nos. 16 and 17: workability in mixing and in adhesive bonding was deteriorated and bond strength was low, because the amount of the ingredient
(D) was too great in contrast to Experiment No. 15.

Experiment No. 18: workability in mixing and in adhesive bonding was deteriorated and bond strength was low, because the amount of the ingredient (F) was too great.

Experiment No. 19: workability in mixing and in adhesive bonding was deteriorated and bond strength was extremely low, because the amount of the ingredient (G) was too great.

As described above, the present invention makes it possible to provide a high-performance dental adhesive composition which can exhibit excellent workability in handling without being adversely affected by water or oxygen contained in an atmosphere when used in adhesive bonding and which can further ensure high bond strength without using a bonding agent (or an adhesive primer), by containing the specific kinds of materials in the specific amounts and containing substantially no radical polymerization initiator.

## Claims

1. A dental adhesive composition comprising:
(A) a carboxylic acid compound having a (meth)-acryloyl group and a carboxyl group, both of which are attached to an aromatic ring, in an amount not smaller than 10% by weight and not greater than 65% by weight;
(B) a bisphenol A derivative having two (meth)-acryloyl groups, in an amount equal to 0% by weight or not greater than 45% by weight; and
(C) a hydroxyalkyl (meth) acrylate in an amount not smaller than 30% by weight and not greater than 70% by weight;
the amounts of the (A), (B), and (C) being all based on the total weight of the (A), (B), and (C);
the composition further comprising:
(D) a (meth)acrylate derivative having an acid group, in an amount not smaller than 10 parts by weight and not greater than 40 parts by weight, based on 100 parts by the total weight of the (A), (B), and (C) ; and
(E) at least one polymerization initiator selected from the group consisting of aromatic amines, aliphatic amines, and aromatic sulfinic acids, in an amount not smaller than 1 part by weight and not greater than 11 parts by weight, based on 100 parts by the total weight of the (A), (B), and (C);
the composition being free from any radical polymerization initiator.

2. The dental adhesive composition according to claim 1, further comprising (F) a filler in an amount smaller than or equal to 350 parts by weight, based on 100 parts by the total weight of the (A), (B), and (C).

3. The dental adhesive composition according to claim 1 or 2, further comprising (G) an oxetane compound in an amount smaller than or equal to 60 parts by weight, based on 100 parts by the total weight of the (A), (B), and (C)

4. The dental adhesive composition according to claim 1, 2 or 3, wherein the carboxylic acid compound (A) is at least one selected from the group consisting of 2-(meth)acryloyloxyethyl hydrogenphthalate and 4-(meth)acryloyloxyethyl hydrogenphthalate.

5. The dental adhesive composition according to anyone of claims 1 to 4, wherein the bisphenol A derivative (B) is at least one selected from the group consisting of 2,2-bis-[4-(3-methacryloyloxy-2-hydroxypropoxy)phenyl]propane and 2,2-bis[(4-methacryloyloxypolyethoxy)phenyl]propane.

6. The dental adhesive composition according to anyone of claims 1 to 5, wherein the (meth)acrylate derivative (D) is at least one selected from the group consisting of 11-methacryloyloxy-1,1-undecanedicarboxylic acid, 4-acryloyloxyethyltrimellitic acid, 10-methacryloyloxydecyl hydrogenphosphate, and 2-methacryloyloxyethylphenyl phosphate.

7. The dental adhesive composition according to anyone of claims 2 to 6, wherein the filler (F) is at least one selected from the group consisting of inorganic fine particles and organic fine particles.

8. The dental adhesive composition according to claim 7, wherein the inorganic fine particles are silica particles having an average particle diameter not smaller than 0.2 µm and not greater than 5 µm.

9. The dental adhesive composition according to anyone of claims 3 to 8, wherein the oxetane compound (G) is at least one selected from the group consisting of 3- (methacryloyloxy (lower alkyl))-3-(lower alkyl) oxetane, tetraphthalate-bisoxetane, and biphenylene-bisoxetane.

## Patentansprüche

1. Dentalklebstoffzusammensetzung, umfassend:
(A) eine Carbonsäureverbindung mit einer (Meth)acryloylgruppe und einer Carboxylgruppe, wobei beide an einen aromatischen Ring gebunden sind, in einer Menge von nicht weniger als 10 Gew.-% und nicht größer als 65 Gew.-%,
(B) ein Bisphenol-A-Derivat mit zwei (Meth)acryloylgruppen, in einer Menge gleich 0 Gew.-% oder nicht größer als 45 Gew.-%, und
(C) ein Hydroxyalkyl(meth)acrylat in einer Menge von nicht weniger als 30 Gew.-% und nicht größer als 70 Gew.-%,
wobei die Mengen von (A), (B) und (C) alle auf das Gesamtgewicht von (A), (B) und (C) bezogen sind,
wobei die Zusammenfassung weiter umfaßt
(D) ein (Meth)acrylatderivat mit einer Säuregruppe in einer Menge von nicht weniger als 10 Gew.-Teilen und nicht größer als 40 Gew.-Teilen, bezogen auf 100 Teile bezüglich des Gesamtgewichts von (A), (B) und (C), und
(E) mindestens einen Polymerisationsinitiator, ausgewählt aus der Gruppe, bestehend aus aromatischen Aminen, aliphatischen Aminen und aromatischen Sulfinsäuren in einer Menge von nicht weniger als 1 Gew.-Teil und nicht größer als 11 Gew.-Teilen, bezogen auf 100 Teile bezüglich des Gesamtgewichts von (A), (B) und (C),
wobei die Zusammensetzung frei von jedwedem radikalischen Polymerisationsinitiator ist.

2. Dentalklebstoffzusammensetzung gemäß Anspruch 1, weiter umfassend
(F) einen Füllstoff in einer Menge von weniger als oder gleich 350 Gew.-Teilen, bezogen auf 100 Teile bezüglich des Gesamtgewichts von (A), (B) und (C).

3. Dentalklebstoffzusammensetzung gemäß Anspruch 1 oder 2, weiter umfassend
(G) eine Oxetanverbindung in einer Menge von weniger als oder gleich 60 Gew.-Teilen, bezogen auf 100 Teile bezüglich des Gesamtgewichts von (A), (B) und (C).

4. Dentalklebstoffzusammensetzung gemäß Anspruch 1, 2 oder 3, wobei die Carbonsäureverbindung (A) mindestens eine, ausgewählt aus der Gruppe, bestehend aus 2-(Meth)acryloyloxethylhydrogenphthalat und 4-(Meth)acryloyloxyethylhydrogenphthalat, ist.

5. Dentalklebstoffzusammensetzung gemäß einem der Ansprüche 1 bis 4, wobei das Bisphenol-A-Derivat (B) mindestens eines, ausgewählt aus der Gruppe, bestehend aus 2,2-Bis-[4-(3-methacryloyloxy-2-hydroxypropoxy)phenyl]-propan und 2,2-Bis[(4-methacryloyloxypolyethoxy)phenyl]propan, ist.

6. Dentalklebstoffzusammensetzung gemäß einem der Ansprüche 1 bis 5, wobei das (Meth)acrylatderivat (D) mindestens eines, ausgewählt aus der Gruppe, bestehend aus 11-Methacryloyloxy-1,1-undecandicarbonsäure, 4-Acryloyloxyethyltrimellithsäure, 10-Methacryloyl-oxydecylhydrogenphosphat und 2-Methacryloyloxy-ethylphenylphosphat, ist.

7. Dentalklebstoffzusammensetzung gemäß einem der Ansprüche 2 bis 6, wobei der Füllstoff (F) mindestens einer, ausgewählt aus der Gruppe, bestehend aus anorganischen feinen Teilchen und organischen feinen Teilchen, ist.

8. Dentalklebstoffzusammensetzung gemäß Anspruch 7, wobei die anorganischen feinen Teilchen Silicateilchen mit einem durchschnittlichen Teilchendurchmesser von nicht weniger als 0,2 µm und nicht größer als 5 µm sind.

9. Dentalklebstoffzusammensetzung gemäß einem der Ansprüche 3 bis 8, wobei die Oxetanverbindung (G) mindestens eine, ausgewählt aus der Gruppe, bestehend aus 3-(Methacryloyl-oxy(niederalkyl))-3-(niederalkyl)oxetan, Tetraphthalat-bisoxetan und Biphenylen-bisoxetan, ist.

## Revendications

1. Une composition adhésive dentaire comprenant :
(A) un composé d'acide carboxylique ayant un groupe (méth)acryloyle et un groupe carboxyle, tous deux attachés à un anneau aromatique, dans une quantité non inférieure à 10% en poids et non supérieure à 65% en poids ;
(B) un dérivé de diphénol A ayant deux groupes (méth)acryloyles, dans une quantité égale à 0% en poids ou non supérieure à 45% en poids ; et
(C) un (méth)acrylate hydroxyalkyle dans une quantité non inférieure à 30% en poids et non supérieure à 70% en poids ;
les quantités des (A), (B) et (C) étant toutes basées sur le poids total des (A), (B), et (C) ;
la composition comprenant en outre:
(D) un dérivé de (méth)acrylate ayant un groupe acide, dans une quantité non inférieure à 10 parts en poids et non supérieure à 40 parts en poids, sur la base de 100 parts en poids total des (A), (B) et (C) ; et
(E) au moins un initiateur de polymérisation sélectionné dans le groupe constitué d'amines aromatiques, d'amines aliphatiques, et d'acides sulfoniques aromatiques, dans une quantité non inférieure à 1 part en poids et non supérieure à 11 parts en poids, sur la base de 100 parts en poids total des (A), (B) et (C) ;
la composition étant libre de tout initiateur de polymérisation radical.

2. La composition adhésive dentaire conformément à la revendication 1, comprenant également (F) une charge dans une quantité inférieure ou égale à 350 parts en poids, sur la base de 100 parts en poids total des (A), (B) et (C).

3. La composition adhésive dentaire conformément à la revendication 1 ou 2, comprenant également (G) un composé d'oxétane dans une quantité inférieure ou égale à 60 parts en poids, sur la base de 100 parts en poids total des (A), (B) et (C).

4. La composition adhésive dentaire conformément à la revendication 1, 2 ou 3, dans laquelle le composé d'acide carboxylique (A) est au moins un sélectionné dans le groupe constitué de phtalate d'hydrogène 2-(méth)acryloyloxyéthyle et de phtalate d'hydrogène 4-(méth)acryloyloxyéthyle.

5. La composition adhésive dentaire conformément à l'une quelconque des revendications 1 à 4, dans laquelle le dérivé de diphénol A (B) est au moins un sélectionné dans le groupe constitué de 2,2-bis-[4-(3-méthacryloyloxy-2-hydroxypropoxy)phényle]propane et de 2,2-bis[(4-méthacryloyloxypolyéthoxy)phényle]propane.

6. La composition adhésive dentaire conformément à l'une quelconque des revendications 1 à 5, dans laquelle le dérivé de (méth)acrylate (D) est au moins un sélectionné dans le groupe constitué de 11-méthacryloyloxy-1,1-acide undécanedicarboxylique, de 4-acide acryloyloxyéthyltrimellitique, de phosphate d'hydrogène 10-méthacryloyloxydécyle, et de phosphate 2-méthacryloyloxyéthylphényle.

7. La composition adhésive dentaire conformément à l'une quelconque des revendications 2 à 6, dans laquelle la charge (F) est au moins un sélectionné dans le groupe constitué de particules fines inorganiques et de particules fines organiques.

8. La composition adhésive dentaire conformément à la revendications 7, dans laquelle les particules fines inorganiques sont des particules de silice ayant un diamètre de particule moyen non inférieur à 0,2 µm et non supérieur à 5 µm.

9. La composition adhésive dentaire conformément à l'une quelconque des revendications 3 à 8, dans laquelle le composé d'oxétane (G) est au moins un sélectionné dans le groupe constitué de 3-(méthylacryloyloxy(alkyle inférieur))-3-(alkyle inférieur)oxétane, de tétraphtalate-dioxétane, et de biphénylène-dioxétane.
